# EUROPÄISCHE PATENTSCHRIFT

(11) **EP 1 972 343 B1**
(45) Veröffentlichungstag und Bekanntmachung des Hinweises auf die Patenterteilung: **12.01.2011**
(21) Anmeldenummer: 08102777.3
(22) Anmeldetag: 19.03.2008
(51) Int. Cl.: A61K 36/73, A61K 36/53, A61P 15/00, A61P 15/02

(54) **Arzneimittel enthaltend Fraumenmantel zur Behandlung von Endometritis, Vaginitis**
Medicine containing lady's mantle for treating endometritis, vaginitis
Médicament comprenant de l'alchémille pour le traitement de l'endométrite, de la vaginite

(30) Priorität: 23.03.2007 DE 102007014595; 05.10.2007 DE 102007048085
(43) Veröffentlichungstag der Anmeldung: 24.09.2008
(73) Patentinhaber: Navalis Nutraceuticals GmbH, 70794 Filderstadt (DE)
(72) Erfinder: Alber, Gabriele, 70794 Filderstadt (DE)
(74) Vertreter: Patentanwälte Ruff, Wilhelm, Beier, Dauster & Partner

(56) Entgegenhaltungen:
- EP-A- 1 194 136
- WO-A-01/11971
- WO-A-2005/092329
- WO-A-2006/082071
- DATABASE WPI Week 200608 Thomson Scientific, London, GB; AN 2006-068431 XP002486764 & CN 1 650 950 A (LANZHOU INST ANIMAL & VETERINARY MEDICIN) 10. August 2005 (2005-08-10)
- LIU J ET AL: "EVALUATION OF ESTROGENIC ACTIVITY OF PLANT EXTRACTS FOR THE POTENTIAL TREATMENT OF MENOPAUSAL SYMPTOMS" JOURNAL OF AGRICULTURAL AND FOOD CHEMISTRY, AMERICAN CHEMICAL SOCIETY. WASHINGTON, US, Bd. 49, Nr. 5, 1. Januar 2001 (2001-01-01), Seiten 2472-2479, XP001068359 ISSN: 0021-8561
- HURTGEN ET AL: "Pathogenesis and treatment of endometritis in the mare: A review" THERIOGENOLOGY, LOS ALTOS, CA, US, Bd. 66, Nr. 3, 1. August 2006 (2006-08-01), Seiten 560-566, XP005556342 ISSN: 0093-691X
- DENNEHY C E: "The Use of Herbs and Dietary Supplements in Gynecology:An Evidence-Based Review" JOURNAL OF MIDWIFERY AND WOMEN'S HEALTH 200611 US, Bd. 51, Nr. 6, November 2006 (2006-11), Seiten 402-409, XP002486763 ISSN: 1526-9523

## Beschreibung

Die Erfindung betrifft ein Arzneimittel oder Nahrungsergänzungsmittel für Mensch und Tier enthaltend Frauenmantel (Alchemilla vulgaris) zur Behandlung und Prophylaxe von Endometritis, Zervizitis der Gebärmuttersowie Vaginitis.

Extrakte von Alchemilla vulgaris sind als Arzneimittel für die Behandlung von Tumorerkrankungen und Angiogenese in EP 975350 beschrieben.

Ferner ist aus EP 1136194 bekannt, dass Alchemilla vulgaris zur Behandlung von Apthen und Hautverletzungen eingesetzt werden kann.

Extrakte von Mönchspfeffer sind beschrieben für das postmenopausale und prämenstruelle Syndrom. Der Mönchspfeffer hat eine hormonregurerende Bedeutung mit Wirkung auf die Regelblutung. Eine dopaminerge Wirkung auf die Hypophyse mit der Folge einer Senkung erhöhter Prolaktinspiegel gilt als gesichert. Durch die Senkung des Prolaktins kommt es zu einer Erhöhung bzw. Normarisierung von Progesteron. Dadurch können Hormonungleichgewichte wie ein Östrogenüberhang normalisiert werden.

Bislang steht kein Arzneimittel oder Nahrungsergänzungsmittel aus pflanzfichen Drogen oder deren Extrakte zur Verfügung, welches zur Behandlung und Prophylaxe von Endometritis, Zervizitis der Gebärmutter sowie Vaginitis bei Mensch und Tier verwendet werden kann.

Ausgehend von diesem Stand der Technik ist es daher Aufgabe der vorliegenden Erfindung, pflanzliche Drogen oder deren Extrakte bereitzustellen, die zur Behandlung und Prophylaxe von Endometritis, Zervizitis der Gebärmutter sowie Vaginitis bei Mensch und Tier verwendet werden können.

Die Lösung dieser Aufgabe erfolgt durch die kennzeichnenden Merkmale in den Ansprüchen.

Überraschender Weise hat sich herausgestellt, dass Alchemilla vulgaris oder Alchemilla vulgaris und Vitex Agnus castus (nachstehend auch: erfindungsgemäße Pflanzendrogen) oder deren Extrakte besonders zur Behandlung und Prophylaxe von Endometritis, Zervizitis der Gebärmutter sowie Vaginitis geeignet ist.

Besonders vorteilhaft wirkt die Pflanzendroge Alchemilla vulgaris und / oder Vitex Agnus castus auf die Regeneration der Schleimhäute der Gebärmutter/Vagina/Vuiva. Bei chronischer Endometritis ist eine Verdikkung der Gebärmutterschleimhaut häufig zu beobachten und kann mittels den erfindungsgemäßen Pflanzendrogen oder deren Extrakte unter Abklingen der Verdickung behandelt werden. Dies geht vorteilhaft mit einer Regeneration der Gebärmutter und der Schleimhäute einher. Ebenfalls für die Einnistung des Konzeptus (Empfängnis) ist eine intakte Gebärmutterschleimhaut Voraussetzung und vorteilhaft mittels der erfindungsgemäßen Pflanzendrogen oder deren Extrakte herzustellen. Weiterhin erfolgt vorteilhaft die Behebung pathogener Schleimhautabsonderungen (Pyometra) mittels der erfindungsgemäßen Pflanzendrogen oder deren Extrakte.

Die erfindungsgemäßen Pflanzendrogen tragen aufgrund ihrer adstringierenden, entzündungs- und sekretionshemmenden Wirkung maßgeblich vorteilhaft zur Regeneration und zur Reduzierung von Flüssigkeitsansammlungen und daher zur Verbesserung der Schleimhautintegrität der Gebärmutter bei.
Die erfindungsgemäßen Pflanzendrogen weisen einen pallativen und curativen Effekt auf.

Vorzugsweise wird die Pflanzendroge Alchemilla vulgaris aus den getrockneten oberen Teilen verwendet. Die wirksamen Inhaltsstoffe sind Gerbstoffe, vor allem Ellagitannine mit dem Hauptinhaltsstoff Agrimoniin, weiterhin Flavonoidglykoside. Eine adstringierende Wirkung dieser Gerbstoffe gilt als gesichert. Bevorzugt werden Blätter und/oder Blütenblätter von Alchemilla vulgaris verwendet.

Bei Vitex Agnus castus werden vorzugsweise die als Steinbeeren bezeichneten getrockneten Früchte verwendet. Die Leitsubstanzen sind die Iridoidglykoside Agnusid und Aucubin sowie Casticin. Darüber hinaus sind ätherische Öle und Diterpene enthalten.

Daher betrifft die Erfindung ebenfalls eine Zusammensetzung oder Zubereitung oder dessen Verwendung einer Zusammensetzung oder Zubereitung enthaltend Alchemilla vulgaris oder enthaltend Alchemilla vulgaris und vitex Agnus castus ggfs. Zusatz und Hilfsstoffe zur Behandlung und Prophylaxe von Endometritis, Zervizitis der Gebärmutter sowie Vaginitis bei Mensch und Tier. Bevorzugt ist die Anwendung der Zusammensetzung / Zubereitung am Säugetier, besonders bevorzugt Huftiere, vorzugsweise Pferd, Haustiere, insbesondere Hund. In einer besonderen Ausführungsform ist die Zubereitung oder Zusammensetzung ein Nahrungsergänzungsmittel oder eine pharmazeutische, vorzugsweise eine veterinärmedizinische Zubereitung oder Zusammensetzung. Ferner betrifft die Erfindung die Verwendung eines solchen Nahrungsergänzungsmittels zur Behandlung und Prophylaxe von Endometritis, Zervizitis der Gebärmutter sowie Vaginitis bei Mensch und Tier.

In einer weiteren Ausführungsform können die erfindungsgemäßen Pflanzendrogen oder deren Extrakte mit anderen Pflanzendrogen oder deren Extrakte der Scharfgarbe (Achillea millefolium) und/oder Blutwurz (Potentilla erecta) vorteilhaft kombiniert werden.

Bei Achillea milleforum werden erfindungsgemäß wahlweise nur die getrockneten Blüten oder die getrockneten Blüten, Blätter und Teile der Stängel verwendet Die wirksamen Inhaltsstoffe dieser polymorphen Art sind vor allem Monoterpene und Sesquiterpene. Neben einer Reihe von Wirkungen sind insbesondere eine entzündungshemmende und antiödematöse Wirkung bestätigt Achillea milleforum wirkt aufgrund der enthaltenen Proazulene ebenfalls entzündungshemmend, darüberhinaus zusätzlich kontraktionsfördernd und antiödematös.

Bei Potentilla erecta werden die getrockneten Wurzelstöcke als Droge verwendet. Die wirksamen Inhaltsstoffe sind ebenfalls Gerbstoffe, vor allem Catechingerbstoffe (Proanthocyanidine) und Ellagitannine. Die Ellagitannine tragen wesentlich zur adstringierenden Wirkung von Potentilla erecta bei. Ähnlich wie bei anderen Gerbstoffdrogen wurden weiterhin antimikrobielle, antiinflammatorische und antioxidative Wirkungen nachgewiesen. Insbesondere kann das Gerbstoffspektrum in der erfindungsgemäßen Kombination durch die Gerbstoffe der Potentilla erecta erweitert und ergänzt werden und führt zu einer Verbesserung der Gesamtwirkung.

Daher betrifft die Erfindung eine solche Zusammensetzung oder Zubereitung oder Kombinationspräparat von Pflanzendrogen oder deren Extrakte, nämlich eine Kombination von Alchemilla vulgaris, Vitex Agnus castus, Achillea milleforum und/oder Potentilla erecta. In einer weiter bevorzugten Ausführungsform ist Alchemilla vulgaris Hauptbestandteil, insbesondere bevorzugt mehr als 34 Gew.%, insbesondere mehr als 51 Gew.%.

Besonders vorteilhaft erlauben die erfindungsgemäßen Kombinationen die besonders wirksame Behandlung und Prophylaxe von Endometritis, Zervizitis der Gebärmutter sowie Vaginitis bei Mensch und Tier.

Die häufig zu beobachtende Ödematisierung des Endometriums kann durch Alchemilla vulgaris und / oder Vitex Agnus castus, vorzugsweise ebenfalls in der Kombinationswirkung von Alchemilla vulgaris und Achillea milleforum verbessert werden. Besonders bevorzugt ist jedoch die Kombination aus Alchemilla vulgaris mit Vitex Agnus castus.

Daher betrifft die Erfindung ebenfalls eine Zusammensetzung oder Zubereitung enthaltend auf 100 Gew.% 2-90 Gew.% Alchemilla vulgaris, oder mindestens 33 Gew.% Alchemilla vulgaris oder mindestens 51 Gew.% Alchemilla vulgaris, ggfs. 2-80 Gew.% Achillea milleforum und/oder 2-50 Gew.% Potentilla erecta; 2-20 Gew.% Vitex Agnus castus, ggfs. 1 - 20 Gew.% weitere Nährstoffe und weitere Hilfsmittel und Zusatzstoffe.

In einer weiteren Ausführungsform der Erfindung besteht die Zubereitung oder Zusammensetzung auf 100 Gew.% aus bis zu 60 Gew.% Alchemilla vulgaris, 10-20 Gew.% Achillea milleforum, 10-20 Gew.% Potentilla erecta und 2 bis 10 Gew.% Vitex Agnus castus und ggfs. auf 100 Gew.% weitere Nährstoffe, Hilfs- und Zusatzstoffe.

In einer weiteren Ausführungsform können die erfindungsgemäßen Zusammensetzungen einem Nahrungsmittel zugesetzt werden. In einer weiteren Ausführungsform können die erfindungsgemäßen Zusammensetzungen einem Tierfutter (Pellets etc.) zugesetzt werden.

In einer bevorzugten Ausführungsform beträgt die Dosierung der erfindungsgemäßen Pflanzendrogen oder Extrakte beim Pferd 2 bis 200 g pro Tag, insbesondere 5 bis 150 g pro Tag, 10-100 g Tag. Beim Menschen und Säugetieren (wie Hund) ist eine Dosierung von 1-50 g pro Tag, insbesondere 2-25 g pro Tag, 1-10 g pro Tag bevorzugt.

Im Rahmen dieser Erfindung wird unter "Endometritis" eine Entzündung der Gebärmutterschleimhaut (Endometrium) verstanden. Die Formen differenzieren sich lokal oder nach Zeitpunkt des Auftretens, insbesondere 1. Endometritis corporis; 2. Endometritis cervicis ("Zervizitis"); 3. Endometritis puerperalis (nach einer Geburt); 4. Endometritis post abortum oder 5. post breeding Endometritis.
Sämtliche Formen einer Endometritis sind erfindungsgemäß umfasst.

Diese Erkrankung tritt häufig auf und bleibt zumeist unerkannt und ist bislang bei chronischem Auftreten schwer zu therapieren. Eine Therapie bei akutem Auftreten beschränkt sich zumeist auf die Antibiose und Uteruslavage, die nur teilweise zum Erfolg führt.

Im Rahmen dieser Erfindung wird unter "Vaginitis" oder synonym Kolpitis eine akute od. chronische Entzündung der Vagina bis hin zum Gebärmutterhals verstanden, häufig zusammen mit Entzündung der Vulva (Vulvovaginitis) infolge zumeist einer bakteriellen Fremdbesiedelung der Vagina.

Zur Herstellung der erfindungsgemäßen Extrakte können übliche organische Lösungsmittel oder Gemische von organischen Lösungsmitteln mit Wasser oder mit überkritischem CO₂ verwendet werden (Alkoholauszug, etc.). Extrakte aus den erfindungsgemäßen Pflanzendrogen können mittels Wasser, Alkohol, Säuren oder Basen, Lösungsmitteln (polar oder unpolar, z.B. Essigsäureethylester o.a.) nach üblichen Verfahren hergestellt werden. Entsprechende Auszüge, Fraktionen können getrocknet werden.

Die Darreichungsform der erfindungsgemäßen Arzneimittel, Zusammensetzung oder Zubereitungen, Kombinationspräparat kann in beliebiger Form für Mensch und Tier erfolgen, wie nicht abschließend flüssig, fest, Tabletten, Teezubereitung, Aufguss, Pulver- oder Pastenform und speziell für die Tierernährung auch in verpresster, pelletierter, extrudierter Form.

Ferner können die Zusammensetzungen oder Zubereitungen, Kombinationspräparate, Formulierungen oder Pellets Nährstoffe wie nicht abschließend Mineralien, Kieselsäure, Vitamine, Proteine samt weiteren Hilfs- und Zusatzstoffe beinhalten.

Die pharmazeutisch wirksamen Mittel können in Form von pharmazeutischen Zubereitungen in Dosierungseinheiten zubereitet werden. Dies bedeutet, dass die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht. Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.
Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt. Tabletten, Dragees, Kapseln, Pillen und Granulate können den oder die Wirkstoffe neben den üblichen Trägerstoffen enthalten, wie a) Füll- und Streckmittel, z.B. Stärken, Milchzucker, Rohrzucker, Glukose, Mannit und Kieselsäure, b) Bindemittel, z.B. Carboxymethylcellulose, Alginate, Gelatine, Polyvinylpyrrolidon, c) Feuchthaltemittel, z.B. Glycerin, d) Sprengmittel, z.B. Agar-Agar, Calciumcarbonat und Natriumcarbonat, e) Lösungsverzögerer, z.B. Paraffin und f) Resorptionsbeschleuniger, z.B. quartäre Ammoniumverbindungen, g) Netzmittel, z.B. Cetylalkohol, Glycerinmonostearat, h) Adsorptionsmittel, z.B. Kaolin und Bentonit und i) Gleitmittel, z.B. Talkum, Calcium- und Magnesiumstearat und feste Polyethylenglykole oder Gemische der unter a) bis i) aufgeführten Stoffe.
Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.
Der oder die Wirkstoffe können gegebenenfalls mit einem odermehreren der oben angegebenen Trägerstoffe auch in mikroverkapselter Form vorliegen.
Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. C14-Alkohol mit C16-Fettsäure) oder Gemische dieser Stoffe.
Salben, Pasten, Cremes und Gele können neben dem oder den Wirkstoffen die üblichen Trägerstoffe enthalten, z.B. tierische und pflanzliche Fette, Wachse, Paraffine, Stärke, Tragant- Cellulosederivate, Polyethylenglykole, Silikone, Bentonite, Kieselsäure, Talkum und Zinkoxid oder Gemische dieser Stoffe.
Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten. Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3- Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.
Die genannten Formulierungsformen können auch Färbemittel, Konservierungsstoffe sowie geruchs- und geschmacksverbessemde Zusätze, z.B. Pfefferminzöl und Eukalyptusöl und Süßmittel, z.B. Saccharin, enthalten.

Die Erfindung wird im Folgenden anhand von Ausführungsbeispielen näher erläutert, ohne die Erfindung auf diese Beispiele zu beschränken:

### Beispiele:

### Beispiel 1

Stute, 21 Jahre, wurde mit Natursprung gedeckt. Nach Bedeckung ist eine drastische Reaktion mit erheblicher Flüssigkeitsansammlung in der Gebärmutter (Pyometra) erfolgt. Erste Behandlungsmaßnahmen wie Antibiose und Uteruslavage führten nur zum kurzzeitigen Erfolg. Die Flüssigkeitsansammlungen kehrten schnell zurück. Neben Ausfluss als deutliches Symptom scheuerte die Stute den Schweif. Ultraschalluntersuchungen zeigen deutliche Verdickungen der Gebärmutterschleimhaut auf. Eine Uterusbiopsie ergab den Befund eines schwer geschädigten Endometriums mit Endometritis und einer bereits bestehenden Endometrose. Ein weiterer Behandlungsversuch mit Homöopathika und Akupunktur ist erfolglos geblieben.
Mit der Supplementierung der erfindungsgemäßen Zusammensetzung wurde begonnen und schrittweise (von Rosse zu Rosse) wurde innerhalb eines Zeitraumes von ca. 3 Monaten die Flüssigkeitsansammlung auf ein Minimum reduziert. Die Stute hat keinen Ausfluss mehr gezeigt und scheuerte sich nicht mehr den Schweif. Es konnte anhand einer Ultraschalluntersuchung gezeigt werden, dass die Verdickungen an der Gebärmutterschleimhaut drastisch verbessert und abgeklungen waren.

### Beispiel 2:

Frau, 46 Jahre, zyklisch wiederkehrende Candida-Vaginitis, üblicherweise ab der Zyklusmitte auftretend. Candida als Begleitkeim und Auslöser der Symptome wie Ausfluss, Juckreiz, etc..
Vorgeschichte: Konventionelle Behandlung über 1 Jahr mit Antimykotika, die jeweils über 6 Tage äußerlich zur Anwendung kommen. Der Erfolg stellt sich immer nur kurzzeitig ein und bereits mit dem nächsten Zyklus kommt es zu einem neuen Infektionsschub. Eine Unterstützung mit Döderlein-Bakterien in die Vagina bringt nur etwas Linderung, kann das Krankheitsgeschehen jedoch nicht unterbrechen.

Nun erfolgt eine tägliche orale Verabreichung der erfindungsgemäßen Zubereitung über 3 Monate ohne Pause. Es kommt bereits im ersten Zyklus nur noch zu einer leichten Candida-Infektion, die bereits nach 3 Monate zur vollständigen Regeneration führt.

### Beispiel 3:

Stute, 16 Jahre, persistierender Corpus luteum, Endometritis.

[0036] Ein Corpus luteum persistens tritt bei Stuten aufgrund unterschiedlicher Ursachen häufig auf. Veränderungen des Endometriums sind eine häufige Ursache, da die für die Luteolyse notwendige Prostaglandinsekretion des geschädigten Endometriums nicht ausreicht. Dies ist eine beim Rind noch regelmäßiger auftretende Erkrankung als beim Pferd. Zu Beginn der Therapie wurde zur Luteolyseinduktion eine PGF_{2α} Applikation durchgeführt. Dann folgte die Verabreichung der erfindungsgemäßen Zubereitung über mehrere Monate. Auch in den Folgejahren kam es nicht wieder zu einem persistierenden Corpus luteum.

## Patentansprüche

1. Alchemilla vulgaris zur Anwendung bei der Behandlung und Prophylaxe von Endometritis, Zervizitis der Gebärmutter sowie Vaginitis bei Mensch und Tier.

2. Alchemilla vulgaris zur Anwendung nach Anspruch 1, **dadurch gekennzeichnet, dass** zusätzlich in einer Zubereitung oder Zusammensetzung Vitex Agnus castus, Achillea milleforum und/oder Potentilla erecta enthalten sind.

3. Alchemilla vulgaris zur Anwendung nach Anspruch 1 oder 2, **dadurch gekennzeichnet, dass** Extrakte aus den Pflanzendrogen verwendet werden.

4. Alchemilla vulgaris zur Anwendung nach einem der Ansprüche 1 bis 3, **dadurch gekennzeichnet, dass** die Darreichungsform flüssig, fest, in Form von Tabletten, Teezubereitung, Aufguß, Pulver- oder Pastenform und speziell für die Tierernährung in verpresster, pelletierter, extrudierter Form erfolgt.

5. Zusammensetzung oder Zubereitung oder Kombinationspräparat enthaltend auf
100 Gew.% 2- 90 Gew.% Alchemilla vulgaris, oder mindestens 33 Gew.% Alchemilla vulgaris oder mindestens 51 Gew.% Alchemilla vulgaris, ggfs. 2-80 Gew.% Achillea milleforum und/oder 2-50 Gew. % Potentilla erecta; 2-20 Gew.% Vitex Agnus castus, ggfs. 1-20 Gew.% weitere Nährstoffe und weitere Hilfsmittel und Zusatzstoffe.

6. Nahrungsergänzungsmittel enthaltend Alchemilla vulgaris oder eine Zusammensetzung nach Anspruch 5 zur Anwendung bei der Behandlung und Prophylaxe von Endometritis, Zervizitis der Gebärmutter sowie Vaginitis bei Mensch und Tier.

7. Arzneimittel enthaltend Alchemilla vulgaris oder eine Zusammensetzung nach Anspruch 5 zur Anwendung bei der Behandlung und Prophylaxe von Endometritis, Zervizitis der Gebärmutter sowie Vaginitis bei Mensch und Tier.

## Claims

1. Alchemilla vulgaris for use in treatment and prophylaxis of endometritis, cervicitis of the uterus as well as vaginitis in humans and animals.

2. Alchemilla vulgaris for use according to claim 1, **characterized in that** in a formulation or composition Vitex agnus castus, Achillea milleforum and/or Potentilla erecta are included additionally.

3. Alchemilla vulgaris for use according to claim 1 or 2, **characterized in that** extracts of vegetable drugs are used.

4. Alchemilla vulgaris for use according to any of claims 1 through 3, **characterized in that** the pharmaceutical form is liquid, solid, in the form of tablets, tea preparations, extractions, in the form of powders or pastes, and in particular for animal feeding in compacted, pelletized and extrudated form.

5. Composition or formulation or combination preparation including in 100 % by weight, 2 through 90 % by weight Alchemilla vulgaris or at least 33 % by weight Alchemilla vulgaris or at least 51 % by weight Alchemilla vulgaris, optionally 2 through 80 % by weight Achillea milleforum and/or 2 through 50 % by weight Potentilla erecta, 2 through 20 % by weight Vitex agnus castus, optionally 1 through 20 % by weight of further nutrients and further adjuvants and additives.

6. Dietary supplement including Alchemilla vulgaris or a composition according to claim 5 for use in treatment and prophylaxis of endometritis, cervicitis of the uterus as well as vaginitis in humans and animals.

7. Medicine including Alchemilla vulgaris or a composition according to claim 5 for use in treatment and prophylaxis of endometritis, cervicitis of the uterus as well as vaginitis in humans and animals.

## Revendications

1. Alchemilla vulgaris pour usage en traitement et prophylaxie de l'endométrite, cervicite de l'utérus ainsi que vaginite dans l'homme ou l'animal.

2. Alchemilla vulgaris pour usage selon la revendication 1, **caractérisée en ce que** dans une formulation ou composition Vitex agnus castus, Achillea milleforum et/ou Potentilla erecta sont inclus additionnellement.

3. Alchemilla vulgaris pour usage selon la revendication 1 ou 2, **caractérisée en ce que** extraits de drogues végétales sont employés.

4. Alchemilla vulgaris pour usage selon l'une des revendications 1 à 3, **caractérisée en ce que** la forme pharmaceutique est liquide, solide, sous forme de tablettes, préparations de tisane, extractions, sous forme de poudre ou pâte, et spécialement en cas d'alimentation pour les animaux sous forme de comprimés, pellets, extrusions.

5. Composition ou formulation ou préparation combinaison comprenant sur 100 % en poids, de 2 à 90 % en poids Alchemilla vulgaris ou au moins 33 % en poids Alchemilla vulgaris ou au moins 51 % en poids Alchemilla vulgaris, optionnellement de 2 à 80 % en poids Alchemilla milleforum et/ou de 2 à 50 % en poids Potentilla erecta, de 2 à 20 % en poids Vitex agnus castus, optionnellement de 1 à 20 % en poids d'autres nutriments et d'autres adjuvants et additives.

6. Supplément nutritionnel comprenant Alchemilla vulgaris ou une composition selon la revendication 5 pour usage en traitement et prophylaxie de l'endométrite, cervicite de l'utérus ainsi que vaginite dans l'homme ou l'animal.

7. Médicament comprenant Alchemilla vulgaris ou une composition selon la revendication 5 pour usage en traitement et prophylaxie de l'endométrite, cervicite de l'utérus ainsi que vaginite dans l'homme ou l'animal.
